# EUROPEAN PATENT SPECIFICATION

(11) **EP 1 298 103 B1**
(45) Date of publication and mention of the grant of the patent: **11.05.2011**
(21) Application number: 01941021.6
(22) Date of filing: 13.06.2001
(51) Int. Cl.: C04B 12/02, A61L 27/12, A61K 6/06, C04B 14/36, C04B 24/00, C04B 28/34, A61L 24/00

(54) **CALCIUM PHOSPHATE CEMENT**
KALZIUMPHOSPHAT ZEMENT
CIMENT EN PHOSPHATE DE CALCIUM

(30) Priority: 26.06.2000 JP 2000190524
(43) Date of publication of application: 02.04.2003
(73) Proprietor: HOYA Corporation, Tokyo 161-8525 (JP)
(72) Inventor: HIRANO, Masahiro, Mitsubishi Materials Corp., Chichibu-gun, Saitama 368-8503 (JP); TAKEUCHI, Hiroyasu, Mitsubishi Materials Corp., Chiyoda-ku, Tokyo 100-8117 (JP)
(74) Representative: Gille Hrabal Struck Neidlein Prop Roos
(86) International application number: PCT/JP2001/004995
(87) International publication number: WO 2002/000565

(56) References cited:
- EP-A- 0 520 690
- JP-A- 6 172 007
- JP-A- 7 033 489
- US-A- 5 180 426
- MIRTCHI A A ET AL: "CALCIUM PHOSPHATE CEMENTS: ACTION OF SETTING REGULATORS ON THE PROPERTIES OF THE -TRICALCIUM PHOSPHATE- MONOCALCIUM PHOSPHATE CEMENTS" 1 November 1989 (1989-11-01), BIOMATERIALS, ELSEVIER SCIENCE PUBLISHERS BV., BARKING, GB, PAGE(S) 634-638 , XP000081742 ISSN: 0142-9612 * abstract *

## Description

### TECHNICAL FIELD

The present invention relates to a calcium phosphate cement that can be used in treatments for reinforcing natural bone in medical fields such as oral surgery, this calcium phosphate cement being capable of forming a high-strength hardened material.

### BACKGROUND ART

Typically, various properties are required of a cement used in treatments for reinforcing natural bone such as: (1) setting properties in which a slurry obtained by adding an aqueous solution for hardening to the cement solidifies; (2) hardening properties in which the solidified material hardens in the presence of moisture; (3) sufficient strength of the hardened material to enable movement of the reinforced bone; and (4) good absorption and replacement in which the hardened material is absorbed by and regenerated into natural bone. Examples of cements having these properties include, for example, a calcium phosphate cement as disclosed in Japanese Application, No. Hei 4-328185, containing 10 to 25 mass% of tetrabasic calcium phosphate, 3 to 10 mass% of dibasic calcium phosphate, and α-tribasic calcium phosphate.

At the same time, the hardened material which is obtained from cements used in treatments for reinforcing natural bone is considered a biologically foreign material. Therefore, it is desirable to obtain a desired amount of strength in the reinforced bone using as little hardened material as possible. Developments have therefore been directed toward improving the strength of the hardened material with the objective of reducing the amount of material employed.

### SUMMARY OF THE INVENTION

The present invention was conceived in view of the above-described circumstances and an object thereof is to provide a calcium phosphate cement that can be employed in treatments for reinforcing natural bone, and is capable of forming a high-strength hardened material.

The present inventors carried out extensive research directed at improving the strength of the hardened material formed from calcium phosphate cement. Specifically, the present invention was completed with the discovery that the strength of the hardened material could be remarkably increased by formulating a hydration product from a hydration reaction between dibasic calcium phosphate and α-tribasic calcium phosphate, blending this hydration product at a proportion of 2 to 10 mass% with respect to the total mass of the calcium phosphate cement, and having this hydration product, which is dispersed in a slurry formed by adding an aqueous solution for hardening to the obtained cement, function as the starting point for the hardening reaction, with the hardening reaction progressing simultaneously centered on this starting point.

In other words, the calcium phosphate cement of the present invention is formed of a mixed composition comprising the product of a hydration reaction between dibasic calcium phosphate and α-tribasic calcium phosphate in an amount of 2 to 10 mass%, tetrabasic calcium phosphate in an amount of 10 to 25 mass%, and dibasic calcium phosphate in an amount of 3 to 10 mass%, with the remainder comprising α-tribasic calcium phosphate and unavoidable impurities.

The blending proportions of the dibasic calcium phosphate and the α-tribasic calcium phosphate in the product obtained from a hydration reaction between the dibasic calcium phosphate and α-tribasic calcium phosphate can have a weight ratio of dibasic calcium phosphate:α-tribasic calcium phosphate = 1:5 to 1:30. The blending proportions of the dibasic calcium phosphate and the α-tribasic calcium phosphate in the product obtained from a hydration reaction between the dibasic calcium phosphate and α-tribasic calcium phosphate may also have a weight ratio of dibasic calcium phosphate:α-tribasic calcium phosphate = 1:10 to 1:20.

### PREFERRED EMBODIMENTS OF THE PRESENT INVENTION

The calcium phosphate cement of the present invention will now be explained more concretely using preferred embodiments thereof.

The calcium phosphate cement of the present invention includes the product of a hydration reaction between dibasic calcium phosphate and α-tribasic calcium phosphate in an amount of 2 to 10 mass%, tetrabasic calcium phosphate in an amount of 10 to 25 mass%, and dibasic calcium phosphate in an amount of 3 to 10 mass%, with the remainder comprising α-tribasic calcium phosphate and unavoidable impurities. It can, for example, be optimally employed in the reinforcement and treatment of natural bone. The calcium phosphate cement of the present invention is not limited to this application solely, however. For example, it may also be used in other applications such as dental cement, plaster alternatives used in dentistry, or construction cement.

The product of a hydration reaction between dibasic calcium phosphate and α-tribasic calcium phosphate (hereinafter, referred to as "hydration product") has the function of further improving the strength of the hardened material as described above. The amount of this hydration product included in the calcium phosphate cement is 2 to 10 mass%, preferably 3 to 8 mass%, and even more preferably 4 to 6 mass% with respect to the total amount of calcium phosphate cement. When the hydration product is included in an amount less than 2 mass%, the desired effect of improving strength tends not to be obtained. In contrast, when the hydration product is included in excess of 10 mass%, the flow qualities of the slurry deteriorate and workability is impaired.

The hydration product can be formulated by mixing dibasic calcium phosphate and α-tribasic calcium phosphate at a mass proportion of dibasic calcium phosphate:α-tribasic calcium phosphate = 1:5 to 1:30, and preferably to form a blended ratio of dibasic calcium phosphate:α-tribasic calcium phosphate = 1:10 to 1:20. Water is then added to the obtained mixture to achieve a ratio of mixture:water = 1:0.2 to 1:0.5, and preferably mixture:water = 1:0.3 to 1:0.4, and the hydration reaction is allowed to proceed. The resulting material is then ground to a particle diameter in the range of 1 to 300 µm.

The tetrabasic calcium phosphate functions to promote absorption and replacement in which the hardened material is regenerated as natural bone. The amount of tetrabasic calcium phosphate included in the calcium phosphate cement is in the range of 10 to 25 mass%, preferably 12 to 22 mass%, and even more preferably 15 to 20 mass% with respect to the total mass of the calcium phosphate cement. When the tetrabasic calcium phosphate is contained in an amount less than 10 mass%, the effect of improved absorption and replacement tends not to occur. On the other hand, when the amount of tetrabasic calcium phosphate exceeds 25 mass%, the strength of the hardened material tends to decrease.

The dibasic calcium phosphate functions to promote setting of the slurry into a hardened material. The amount of dibasic calcium phosphate included in the calcium phosphate cement is in the range of 3 to 10 mass%, preferably 3.5 to 8 mass%, and even more preferably 4 to 6 mass%. When the dibasic calcium phosphate is contained in an amount less than 3 mass%, the effect of promoting the desired setting tends not to be obtained. On the other hand, when the amount of dibasic calcium phosphate exceeds 10 mass%, the hardening time becomes too short and workability unavoidably deteriorates.

Thus, the calcium phosphate cement of the present invention can be formulated by blending the hydration product of dibasic calcium phosphate and α-tribasic calcium phosphate in an amount of 2 to 10 mass%, tetrabasic calcium phosphate in an amount of 10 to 25 mass%, dibasic calcium phosphate in an amount of 3 to 10 mass%, and α-tribasic calcium phosphate.

### TESTS

Next, the calcium phosphate cement of the present invention will be explained more concretely using tests.

First, the hydration product, α-tribasic calcium phosphate, and tetrabasic calcium phosphate are formulated as described below. A commercial product is used for the dibasic calcium phosphate.

### (1) α-tribasic calcium phosphate

3 moles of calcium hydroxide were suspended in 10 liters of water.
Approximately 0.5 liters of a 40 mass% aqueous phosphoric acid solution formed by diluting 2 moles of phosphoric acid with water were slowly dripped into this suspension while stirring. Once the drop-wise addition was complete, the obtained suspension was left undisturbed at room temperature for one day. A dryer was then employed to dry the suspension for 24 hours at 110°C, to obtain an aggregate. This aggregate was then baked for 3 hours at 1400°C. The baked product was then ground and a screen was used to obtain particles that were 88 µm or less (average particle size: 6.5 µm) in size, to form a 99.9 mass% pure α-tribasic calcium phosphate.

### (2) Tetrabasic calcium phosphate

4 moles of calcium hydroxide were suspended in 10 liters of water.
Approximately 0.5 liters of a 40 mass% aqueous phosphoric acid solution formed by diluting 2 moles of phosphoric acid with water were slowly dripped into this suspension while stirring. Once the drop-wise addition was complete, the obtained suspension was left undisturbed at room temperature for one day. A dryer was then employed to dry the suspension for 24 hours at 110°C, to obtain an aggregate. Next, the aggregate was prebaked for 3 hours at 900°C, after which the material was uniformly ground. Baking was then completed by maintaining the material at 1400°C for 3 hours. The obtained baked product was then ground and a screen was used to obtain particles that were 88 µm or less (average particle size: 6.5 µm) in size, to form a mixed product containing tetrabasic calcium phosphate and hydroxy apatite which is an unavoidable impurity. Note that in this example, the aforementioned mixed product is employed as tetrabasic calcium phosphate.

### (3) Hydration product

The α-tribasic calcium phosphate formulated in (1) above and a commercially available dibasic calcium phosphate were mixed at the blending ratios shown in Table 1. Water was added to this mixture at a weight ratio of mixture:water = 3:1 and the mixture was hardened. The hardened material was ground to a size of 300 µm or less, to formulate hydration products (a) to (f).

Next, the α-tribasic calcium phosphate obtained in (1) to (3) above, tetrabasic calcium phosphate, hydration products (a) to (f), and commercially available dibasic calcium phosphate were employed as raw powders. These raw powders were mixed at the blending ratios shown in Table 2 to obtain the cements 1 to 12 of the present invention which are the preferred embodiments of this invention, and conventional cements 1 to 5 which are examples of the conventional art.

The strength of the hardened material formed from cements 1 to 12 of the present invention and conventional cements 1 to 5 was measured using the following method and was evaluated. First, an aqueous solution for hardening containing 5 mass% sodium chondroitin sulfate, 20 mass% disodium succinate hexahydrate and water was added to each of the cements at a mass ratio of cement:aqueous solution for hardening = 3:1, and ground to obtain a slurry. This slurry was set, forming a cylindrical aggregate having a diameter of 7 mm and a length of 14 mm. This aggregate was hardened by soaking for three days in an aqueous solution simulating body fluid that contained Na⁺: 142.0 mM, K⁺: 5.0 mM, Mg²⁺: 1.5 mM, Ca²⁺: 2.5 mM, Cl⁻:148.8 mM, HCO₃⁻:4.2 mM, and HPO₄²⁻: 1.0 mM. The compression strength of the obtained hardened material was measured and these results are shown in Table 2.

**Table 1**

| | | **Blending ratio (mass ratio)** | |
|---|---|---|---|
| **Category** | | **Dibasic calcium phosphate** | **α-tribasic calcium phosphate** |
| Hydration product | (a) | 1 | 5 |
| | (b) | 1 | 10 |
| | (c) | 1 | 15 |
| | (d) | 1 | 20 |
| | (e) | 1 | 25 |
| | (f). | 1 | 30 |

**Table 2**

| | | **Blending ratio (mass %)** | | | | **Compression strength (MPa)** |
|---|---|---|---|---|---|---|
| **Category** | | **Hydration product** | **Tetrabasic calcium phosphate** | **Dibasic calcium phosphate** | **α-tribasic calcium phosphate** | |
| Cement of the present invention | 1 | (a):5 | 18 | 5 | balance | 76 |
| | 2 | (b):5 | 18 | 5 | balance | 79 |
| | 3 | (c):5 | 18 | 5 | balance | 86 |
| | 4 | (d):5 | 18 | 5 | balance | 80 |
| | 5 | (e):5 | 18 | 5 | balance | 82 |
| | 6 | (f):5 | 18 | 5 | balance | 88 |
| | 7 | (c):2 | 18 | 5 | balance | 70 |
| | 8 | (c):10 | 18 | 5 | balance | 81 |
| | 9 | (c):5 | 10 | 5 | balance | 78 |
| | 10 | (c):5 | 25 | 5 | balance | 71 |
| | 11 | (c):5 | 18 | 3 | balance | 83 |
| | 12 | (c):5 | 18 | 10 | balance | 74 |
| Conventional cement | 1 | - | 10 | 5 | balance | 61 |
| | 2 | - | 18 | 5 | balance | 60 |
| | 3 | - | 25 | 5 | balance | 57 |
| | 4 | - | 18 | 3 | balance | 58 |
| | 5 | - | 18 | 10 | balance | 52 |

The results shown in Table 2 demonstrate that the hardened material formed using cements 1 to 12 of the present invention, in which the product obtained from a hydration reaction between α-tribasic calcium phosphate and dibasic calcium phosphate was added to a mixed composition, had a higher degree of strength than the hardened material formed using conventional cements 1 to 5 which did not contain this hydration product.

### INDUSTRIAL APPLICABILITY

The calcium phosphate cement of the present invention enables formation of a hardened material which is very strong, and has properties useful in industrial applications such as greatly contributing to an improvement in the strength of bone subjected to reinforcing treatment, and reducing the amount of the hardened material employed.

## Claims

1. A calcium phosphate cement comprising a mixed composition comprising the product of a hydration reaction between dibasic calcium phosphate and α-tribasic calcium phosphate in an amount of 2 to 10 mass%, tetrabasic calcium phosphate in an amount of 10 to 25 mass%, and dibasic calcium phosphate in an amount of 3 to 10 mass%, with the remainder comprising α-tribasic calcium phosphate and unavoidable impurities.

2. A calcium phosphate cement according to claim 1, in which the blending proportions of the dibasic calcium phosphate and the α-tribasic calcium phosphate in said product obtained from a hydration reaction between the dibasic calcium phosphate and α-tribasic calcium phosphate have a mass ratio of dibasic calcium phosphate:α-tribasic calcium phosphate = 1:5 to 1:30.

3. A calcium phosphate cement according to claim 1, in which the blending proportions of the dibasic calcium phosphate and the α-tribasic calcium phosphate in said product obtained from a hydration reaction between the dibasic calcium phosphate and α-tribasic calcium phosphate is a mass ratio of dibasic calcium phosphate:α-tribasic calcium phosphate = 1:10 to 1:20.

## Patentansprüche

1. Calciumphosphat-Zement, umfassend eine Mischzusammensetzung, umfassend das Produkt einer Hydratisierungsreaktion zwischen dibasischem Calciumsulfat und α-tribasischem Calciumphosphat in einer Menge von 2 bis 10 Gew.-%, tetrabasisches Calciumphosphat in einer Menge von 10 bis 25 Gew.-%, und dibasisches Calciumphosphat in einer Menge von 3 bis 10 Gew.-%, wobei der Rest α-tribasisches Calciumphosphat und unvermeidliche Verunreinigungen umfasst.

2. Calciumphosphat-Zement nach Anspruch 1 , wobei die Mischungsverhältnisse des dibasischen Calciumphosphats und des αtribasischen Calciumphosphats im Produkt, das aus der Hydratisierungsreaktion zwischen dem dibasischen Calciumphosphat und α-tribasischem Calciumphosphat erhalten wird, ein Gewichtsverhältnis von dibasischem Calciumphosphat : α-tribasischem Calciumphosphat = 1 : 5 bis 1 : 30 zeigen.

3. Calciumphosphat-Zement nach Anspruch 1, wobei die Mischungsverhältnisse des dibasischen Calciumphosphats und des αtribasischen Calciumphosphats im Produkt, das aus der Hydratisierungsreaktion zwischen dem dibasischen Calciumphosphat und α-tribasischem Calciumphosphat erhalten wird, ein Gewichtsverhältnis von dibasischem Calciumphosphat : α-tribasischem Calciumphosphat = 1 : 10 bis 1 : 20 ist.

## Revendications

1. Ciment de phosphate de calcium, comprenant une composition mixte comprenant le produit d'une réaction d'hydratation entre de l'hydrogénophosphate de calcium et de l'orthophosphate de calcium α en une quantité de 2 à 10 % en masse, du pyrophosphate de calcium en une quantité de 10 à 25 % en masse, et de l'hydrogénophosphate de calcium en une quantité de 3 à 10 % en masse, le reste comprenant de l'orthophosphate de calcium α et des impuretés inévitables.

2. Ciment de phosphate de calcium selon la revendication 1, dans lequel les proportions de mélange de l'hydrogénophosphate de calcium et de l'orthophosphate de calcium α dans ledit produit obtenu à partir d'une réaction d'hydratation entre l'hydrogénophosphate de calcium et l'orthophosphate de calcium α, correspondent à un rapport massique hydrogénophosphate de calcium : orthophosphate de calcium α = 1 : 5 à 1 : 30.

3. Ciment de phosphate de calcium selon la revendication 1, dans lequel les proportions de mélange de l'hydrogénophosphate de calcium et de l'orthophosphate de calcium α dans ledit produit obtenu à partir d'une réaction d'hydratation entre l'hydrogénophosphate de calcium et l'orthophosphate de calcium α, correspondent à un rapport massique hydrogénophosphate de calcium : orthophosphate de calcium α = 1 : 10 à 1 : 20.
